# EUROPEAN PATENT SPECIFICATION

(11) **EP 3 053 552 B1**
(45) Date of publication and mention of the grant of the patent: **30.05.2018**
(21) Application number: 14848415.7
(22) Date of filing: 24.09.2014
(51) Int. Cl.: A61B 17/34, A61F 9/007

(54) **CANNULA**
KANÜLE
CANULE

(30) Priority: 30.09.2013 JP 2013203399
(43) Date of publication of application: 10.08.2016
(73) Proprietor: MANI, INC., Utsunomiya-shi, Tochigi 321-3231 (JP)
(72) Inventor: MURAKAMI, Etsuo, Utsunomiya-shi Tochigi 321-3231 (JP); OGANE, Kaoru, Utsunomiya-shi Tochigi 321-3231 (JP)
(74) Representative: Isarpatent
(86) International application number: PCT/JP2014/075177
(87) International publication number: WO 2015/046192

(56) References cited:
- EP-A1- 0 216 952
- WO-A1-2009/120075
- WO-A1-2010/064062
- WO-A1-2013/047473
- JP-A- H08 504 106
- JP-A- 2008 531 077
- JP-A- 2009 219 879
- JP-A- 2011 515 185
- JP-A- 2013 515 563
- US-A- 5 540 699
- US-A1- 2006 089 526
- US-A1- 2008 195 044
- US-A1- 2009 030 443
- US-A1- 2011 152 774
- US-B1- 7 846 134

## Description

### [Technical Field]

The present invention relates to a cannula used in ophthalmic operations.

### [Background Art]

The cannula is used when using a surgical tool or the like within an eyeball during an ophthalmic operation (e.g., Patent Document 1). FIG. 3 is a diagram illustrating a state of an ophthalmic operation. A conventional cannula 110 is configured by fitting a metal pipe 111 into a base 112 made of hard resin. The base 112 has a nearly cylindrical shape and a groove 112a formed along the circumference near the middle position of its side surface so that the cannula 110 can be held by forceps.

If the cannula 110 is pierced through an eyeball A, the base 112 acts as a stopper by making contact with the eyeball A, keeping the cannula in a pierced state. A variety of surgical instruments 20 and an optical instrument for monitoring etc. are then inserted in the eyeball via the cannula 110. As a result, three or four of the cannula 110 may be pierced through an eyeball during surgery.

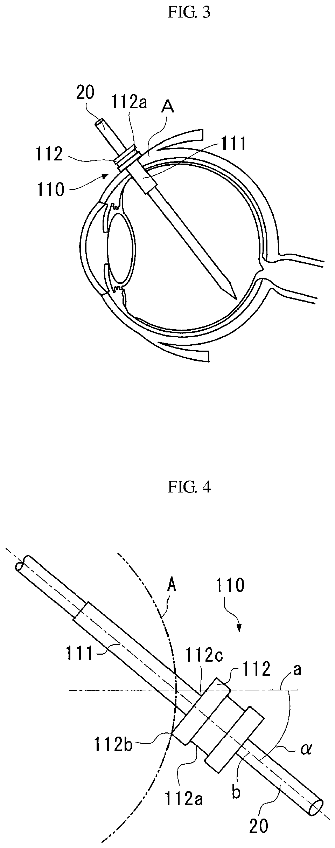

FIG. 4 is a diagram illustrating a state where a conventional cannula is pierced through an eyeball. If the surgical tool 20 is moved while the cannula is in the pierced state, an angle α of the axis b of the cannula 110 and the normal direction a of the eyeball A may be around 30 to 45 degrees, as shown in the drawing.

At this time, in the case of the base 112 made of hard resin and having a nearly cylindrical shape, if the surgical tool 20 is moved to change the angle α of the cannula 110, an angular part 112b of the base 112 makes contact with the eyeball A, and thus a problem that the portion where the pipe 111 pierces through becomes shallow such that the cannula 110 may slip out from the eyeball A during surgery may occur. In addition, a problem that the larger the angle α becomes the greater the force grows in the direction of separating the pipe 111 from the junction 112c of the base 112 with the angular part 112b as a fulcrum, making it easy for the pipe 111 to detach from the base 112, may also occur.

Moreover, the contact place of the eyeball A and the base 112 is a point of contact on the angular part 112b, thereby allowing the base 112 to roll from side to side on the eyeball A. As a result, there is also a problem that the eyeball A may be damaged when the base 112 is made of hard resin.

### [Prior Art Documents]

### [Patent Documents]

Patent Document 1: WO 2010/126076A

WO 2009/120075 A1, which is considered to present the prior art closest to the subject-matter of claim 1, discloses an eye surgery instrument, comprising an entry module provided with a sharp distal end. Furthermore, the instrument comprises an operating element for manipulating the eye surgery instrument. The operating element is attached to the entry module via a curved segment. The curved segment preferably comprises a kink.

WO 2013/047473 A1 discloses A trocar cannula provided with a base and a hollow pipe-shaped cannula of a lesser diameter than the base, the cannula being erected on the base, wherein a slope having an obtuse angle with the center axis of the cannula is formed on the base.

US 7846134 B1 discloses a flexible walled cannula apparatus and method of use comprising a uniquely deformable cannula tube and head in combination with a uniquely shaped obturator which allows the use of heretofore unusable larger gauge surgical instruments while providing a self sealing incision. The apparatus and method of use provides a preassembled obturator and cannula assembly with which the surgeon forms an incision or channel, inserts the cannula tube, and through the cannula inserts surgical instruments to perform a surgical operation. The apparatus and method of use is especially suited for ophthalmic surgical operations. Alternative embodiments for use with even larger diameter instruments utilize a uniquely designed valve having leaflets which prevent bodily or other fluid leakage from the cannula when an instrument is not inserted.

### [DISCLOSURE OF INVENTION]

### [Problem to be Solved by the Invention]

In light of these conditions, the present invention aims to provide a cannula that is capable of controlling itself from easily slipping out from the eyeball when a surgical tool or the like is moved, does not damage the eyeball even when the base of the cannula and the eyeball make contact, and controls the pipe from detaching from the base.

### [Solution to the Problem]

According to an aspect of the present invention, a cannula for piercing through an eyeball in ophthalmic operations having the features of claim 1 is provided.

Moreover, the center part of an eyeball side end surface of the base is depressed, and the shape of the contact portion of the base is changed when the base makes contact with the eyeball.

### [Advantageous Effect of the Invention]

According to the present invention, since the base has flexibility, there are beneficial effects that the eyeball is not damaged even if a surgical tool or the like is moved, and the pipe is not easily detached from the base. Moreover, since the base changes its own shape, there are beneficial
effects that thickness of the pipe pierced through the eyeball hardly changes, and the cannula is controlled from easily slipping out.

### [BRIEF DESCRIPTION OF DRAWINGS]

FIG. 1 is a top view of a cannula of the present invention;
FIG. 2 illustrates states in which the cannula of the present invention is pierced through an eyeball and moved; where 2(a) shows a state where the cannula has been moved a little, and 2(b) shows a state where the cannula has been moved a lot;
FIG. 3 is a diagram illustrating a state of an ophthalmic operation; and
FIG. 4 is a diagram illustrating a state where a conventional cannula is pierced through an eyeball.

### [DESCRIPTION OF EMBODIMENTS]

An embodiment according to the present invention is described below with reference to accompanying drawings.

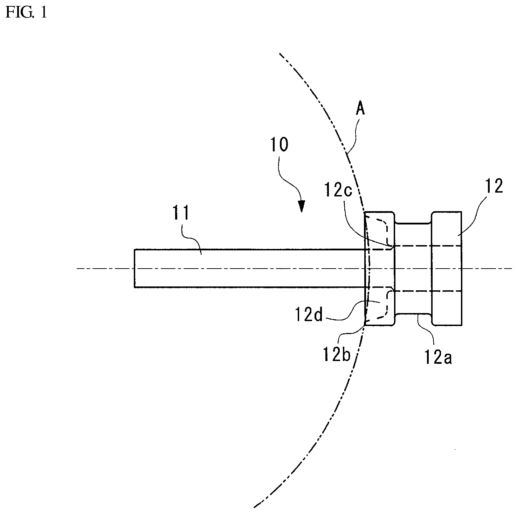

FIG. 1 is a top view of a cannula of the present invention. A cannula 10 of the present invention is constituted by a metal pipe 11 and a nearly cylindrical base 12. An end side of the pipe 11 is pierced through the center of the cylindrical base 12 and attached thereto. The base 12 acts as a stopper when the pipe 11 is pierced through an eyeball A.

The base 12 has a groove 12a formed along the circumference on the cylindrical side surface. This groove 12a is used in holding the cannula with forceps. Moreover, an end of the base 12 on the side in contact with the eyeball A has a form having a depression 12d near the center of the circle. That is, the eyeball side of a cross-section cut along the length of the base 12 has a concave shape. Furthermore, since the depression 12d will fit the curved surface of the eyeball A, the eyeball A and the base 12 are easily attached to each other, as shown in FIG. 1.

The base 12 has flexibility (elasticity); more specifically, it should be made of a more flexible material than the eyeball A. If the base 12 has flexibility, the surgical tool can be moved while the eyeball A and the base 12 are in an attached state, as shown in FIG. 1.

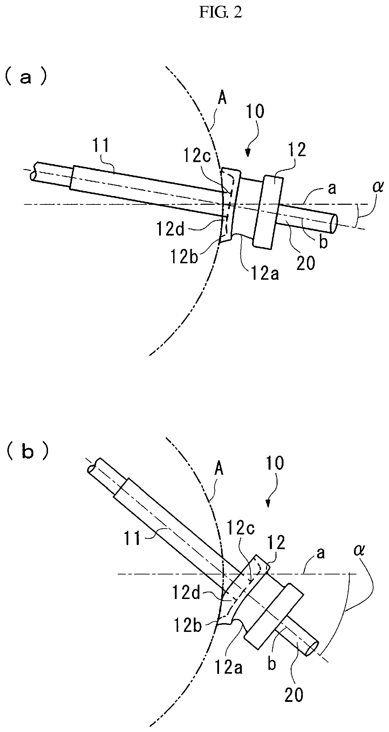

FIG. 2 illustrates states in which the cannula of the present invention is pierced through an eyeball and moved; where 2(a) shows a state where the cannula was moved a little, and 2(b) shows a state where the cannula was moved a lot. The state where the cannula was moved a little, shown in FIG. 2(a) is a state where the cannula 10 is moved resulting from moving the surgical tool 20 while the eyeball A and the base 12 are in a nearly attached state. The state where the cannula was moved a lot, shown in FIG. 2(b) is a state where, as a result of moving the surgical tool 20, an angle α of the normal direction a of the eyeball A and the axis b of the cannula 10 is 30 to 45 degrees.

Since the base 12 has flexibility, if the cannula 10 is pressed against the eyeball A particularly when the base 12 is more flexible than the eyeball A, the shape of the base 12 is changed in conformity with the shape of the eyeball A, as shown in FIG. 2(a) FIG. and 2(b). At this time, if the base 12 has the depression 12d at the central part of the eyeball side end, flexibility further increases and its shape will thus be more easily changed.

Moreover, if the shape of an angular part 12b of the base 12 is changed when the base 12 and the eyeball A make contact, there is a beneficial effect that the eyeball A is not easily damaged. Furthermore, if the shape of the angular part 12b is changed, force does not generate in the detaching direction of the junction of pipe 11 and the base 12 as conventionally, but rather the base 12 is pressed against the pipe 11, and thus there is an effect of keeping the pipe 11 from detaching from the base 12.

Note that the manufacturing method of the base 12 may adopt, for example, injection molding by pouring two-part liquid silicone into a mold and cooling it. That is, since even when the base has flexibility, the cannula of the present invention may be manufactured by the same process as the conventional method, there is no disadvantage in manufacturing.

### [Explanation of References]

10: Cannula
11: Pipe
12: Base
12a: Groove
12b: Angular part
12c: Junction
12d: Depression
20: Surgical tool
A: Eyeball

## Claims

1. A cannula (10) for piercing through an eyeball in ophthalmic operations, comprising:
a metal pipe (11) for piercing through the eyeball; and
a base (12) joined to an end side of the pipe (11), wherein a center part (12d) of the eyeball side end surface of the base (12) has a concave shape configured to fit a curved surface of the eyeball (A), wherein the base (12) is more flexible than the eyeball such that a shape of a contact portion of the base (12) is changed when the base (12) makes contact with the eyeball.

## Patentansprüche

1. Kanüle (10) zum Durchstechen eines Augapfels in ophthalmischen Operationen, umfassend:
ein Metallrohr (11) zum Durchstechen des Augapfels und eine Basis (12), die mit einer Endseite des Rohrs (11) verbunden ist,
wobei ein Mittelteil (12d) der Augapfel-seitigen Endfläche der Basis (12) eine konkave Form hat, die ausgestaltet ist, um zu einer gekrümmten Fläche des Augapfels (A) zu passen,
wobei die Basis (12) derart flexibler ist als der Augapfel, dass eine Form eines Kontaktabschnitts der Basis (12) geändert wird, wenn die Basis (12) mit dem Augapfel in Kontakt kommt.

## Revendications

1. Canule (10) destinée à percer un globe oculaire lors d'opérations ophtalmologiques, comprenant :
un tube métallique (11) destiné à percer le globe oculaire ; et
une base (12) reliée à un côté d'extrémité du tube (11), dans laquelle une partie centrale (12d) de la surface d'extrémité côté globe oculaire de la base (12) présente une forme concave conçue pour s'adapter à une surface courbe du globe oculaire (A),
dans laquelle la base (12) est plus flexible que le globe oculaire, de sorte qu'une forme de partie de contact de la base (12) se modifie lorsque la base (12) entre en contact avec le globe oculaire.
